# EUROPEAN PATENT SPECIFICATION

(11) **EP 2 389 973 B1**
(45) Date of publication and mention of the grant of the patent: **16.10.2013**
(21) Application number: 11164569.3
(22) Date of filing: 03.05.2011
(51) Int. Cl.: A61M 25/00, A61M 25/10

(54) **Balloon catheter**
Ballonkatheter
Cathéter à ballonnet

(30) Priority: 25.05.2010 JP 2010118918
(43) Date of publication of application: 30.11.2011
(73) Proprietor: ASAHI INTECC CO., LTD., Nagoya-shi, Aichi 463-0024 (JP)
(72) Inventor: Iwamuro, Shigenobu, Nagoya-shi Aichi 463-0024 (JP); Ikegaya, Michihiro, Nagoya-shi Aichi 463-0024 (JP); Katsurada, Takeharu, Nagoya-shi Aichi 463-0024 (JP)
(74) Representative: Winter, Brandl, Fürniss, Hübner, Röss, Kaiser, Polte - Partnerschaft

(56) References cited:
- WO-A2-2006/135581
- US-A1- 2007 088 380
- US-A1- 2010 004 593

## Description

### Technical Field

The present invention relates to a balloon catheter used to dilate a stenosis or the like inside a body cavity such as a vessel.

### Background Art

Conventionally, a balloon catheter is used to dilate a stenosis or the like inside a body cavity such as a vessel. The balloon catheter mainly includes a balloon to be dilated, an outer shaft, and an inner shaft arranged inside the outer shaft. The inner shaft is for enabling a guidewire to be inserted therethrough. The outer shaft is used to flow, through a lumen, liquid such as a contrast agent or saline for dilating the balloon. The lumen is provided between the inner shaft and the outer shaft.

Such a balloon catheter is inserted into a vessel or the like and positioned at a desired site. For the positioning, the balloon catheter is operated by an operator such as a physician, in such a manner that torque applied from a proximal side is transmitted to a front end of the catheter. The transmitted torque mainly acts as the force to push the catheter in its axial direction, i.e., so-called pushing force. The balloon catheter is required to have high torque transmissibility of the pushing force.

The balloon catheters conventionally proposed to improve such torque transmissibility include the one in which a braiding is interposed in a shaft (see, for example, JP-A-2001-157712), and the one using a stranded coil formed of a plurality of the strand for a shaft (see, for example, U.S. Patent Application Publication Nos. 2008/0287786 and 2006/0142704).

The use of the above-described braiding or stranded coil is considered effective, to some extent, to improve the torque transmissibility of the balloon catheter. However, the braiding or stranded coil provided on an outer circumference of the inner shaft makes it difficult to sufficiently transmit the torque applied from the outer shaft. When the braiding or stranded coil is used as the outer shaft, on the other hand, the balloon attached to the front end of the outer shaft blocks the torque from the outer shaft. In this manner, there has been a problem in that the torque cannot sufficiently be transmitted to the front end of the catheter.

In addition, when the balloon catheter is to advance toward a target site along the guidewire, the advance of the balloon catheter may be blocked at a tortuous part or the like of a vessel. The present inventors have thought that, even when such a problem arises, the advance of the balloon catheter will be much facilitated if the front end thereof can be turned a predetermined amount around the axis. With this in mind, the present inventors have attempted to improve not only the pushing force in the conventional technique but also the force to turn the front end of the catheter (also called rotational force) when the catheter is turned by a predetermined angle around the axis at a proximal side.

Further, WO 2006/135581 A2 discloses a balloon catheter according to the preamble of claim 1. In particular, WO 2006/135581 A2 discloses a balloon cathether having an inner tubular member, a balloon defining an inflation cavity and an outer tubular member, with a closely wound coil being disposed on the inner tubular member in the balloon cavity. The coil may be attached at a proximal end to the outer tubular member and at a distal end to the distal waist of the balloon. The coil is mentioned to provide columnar support to prevent distal movement of the outer tubular member from compressing the balloon while permitting the balloon to expand. The coil may be a spiral cut tube, a shaped wire coil or other suitable configuration. In one embodiment the coil is also distally attached to the inner tubular member.

### Summary of invention

An object of the invention is to provide a balloon catheter capable of sufficiently transmitting, to a front side of a balloon, two types of torque, i.e., the pushing force and the rotational force which are applied to the balloon catheter from the proximal side.

Note that the "torque transmissibility" mentioned in this specification refers to both the transmissibility of the pushing force and that of the rotational force, unless either one of them is specified.

The above object of the present invention is achieved by a balloon catheter according to claim 1.

A balloon catheter according to the present invention includes: a balloon; a tubular outer shaft connected to a rear end attachment part of the balloon; an inner shaft inserted into the outer shaft and having an extension part extending from a front end of the outer shaft into the balloon, the extension part being connected to a front end attachment part of the balloon; a large-diameter coil part including at least one strand wound around at least a part of the outer shaft; a small-diameter coil part including at least one strand wound around at least a part of the extension part of the inner shaft; and a transitional coil part including at least one strand which connects the large-diameter coil part and the small-diameter coil part, and having a supply hole through which liquid for dilating the balloon is supplied.

Advantageous embodiments of the balloon catheter are subject-matter of dependent claims.

### Brief Description of Drawings

The foregoing and other objects, features, aspects and advantages of the invention will become more apparent from the following detailed description when taken in conjunction with the accompanying drawings.
Fig. 1 is a view illustrating an entire balloon catheter according to the present embodiment.
Fig. 2 is an enlarged view illustrating a front portion of the balloon catheter according to the present embodiment.
Fig. 3 is an enlarged view of the portion D of Fig. 1.
Fig. 4 is a cross-sectional view as seen in the direction of IV-IV of Fig. 1.
Fig. 5 is a cross-sectional view as seen in the direction of V-V of Fig. 1.
Fig. 6 is a cross-sectional view as seen in the direction of VI-VI of Fig. 1.

### Description of Embodiments

Preferred embodiments of the present invention will be described below with reference to the accompanying drawings, in which like reference characters designate similar or identical parts throughout the several views thereof.
<1 > A balloon catheter according to a first aspect of the present invention includes: a balloon; a tubular outer shaft connected to a rear end attachment part of the balloon; an inner shaft inserted into the outer shaft and having an extension part extending from a front end of the outer shaft into the balloon, the extension part being connected to a front end attachment part of the balloon; a large-diameter coil part including at least one strand wound around at least a part of the outer shaft; a small-diameter coil part including at least one strand wound around at least a part of the extension part of the inner shaft; and a transitional coil part including at least one strand which connects the large-diameter coil part and the small-diameter coil part, and having a supply hole through which liquid for dilating the balloon is supplied.
<2> A second aspect of the present invention is the balloon catheter according to the first aspect, wherein each strand of the large-diameter coil part, the small-diameter coil part, and the transitional coil part is formed of a single, seamless strand.
<3> A third aspect of the present invention is the balloon catheter according to the first or second aspect, wherein the large-diameter coil part, the small-diameter coil part, and the transitional coil part are formed of a stranded coil including a plurality of strands.
<4> A fourth aspect of the present invention is the balloon catheter according to any one of the first to third aspects, wherein the large-diameter coil part is arranged at a front side of the outer shaft, and a rear side of the outer shaft is formed of a metal tubular member.
<5> A fifth aspect of the present invention is the balloon catheter according to any one of the first to fourth aspects, wherein an interval between adjacent coils of the strand constituting the small-diameter coil part is larger than an interval between adjacent coils of the strand constituting the large-diameter coil part.
<6> A sixth aspect of the present invention is the balloon catheter according to the fifth aspect, wherein the interval between the adjacent coils of the strand constituting the small-diameter coil part becomes larger toward a front end of the extension part of the inner shaft.
<7> A seventh aspect of the present invention is the balloon catheter according to any one of the first to sixth aspects, wherein coils of the strand constituting the small-diameter coil part reach a front end portion of the extension part of the inner shaft, the front end portion of the extension part being connected to the front end attachment part of the balloon.
<8> An eighth aspect of the present invention is the balloon catheter according to any one of the first to seventh aspects, wherein an interval between adjacent coils of the strand constituting the transitional coil part is larger than an interval between adjacent coils of the strand constituting the large-diameter coil part, and the interval formed between the adjacent coils of the strand in the transitional coil part forms the supply hole.

<1> In the balloon catheter according to the first aspect of the present invention, the large-diameter coil part is provided in the outer shaft, which can improve the torque transmissibility. In addition, the small-diameter coil part is provided at the extension part of the inner shaft, and the large-diameter coil part and the small-diameter coil part are connected by the transitional coil part. With this configuration, the pushing force or rotational force, which is transmitted from the outer shaft by an operator such as a physician, is transmitted to the extension part of the inner shaft, and further to the front end of the balloon catheter. Therefore, the balloon does not block the transmission of the torque from the outer shaft, making it possible to exhibit high torque transmissibility.
   Therefore, the front end of the balloon catheter can suitably be turned around the axis thereof. This facilitates the advance of the balloon catheter in a vessel, even when the advance of the front end of the balloon catheter is blocked once at a tortuous part or the like of the vessel.
   In addition, in the balloon catheter according to the first aspect, the transitional coil part, which connects the large-diameter coil part and the small-diameter coil part, has the supply hole through which the liquid for dilating the balloon is supplied. Therefore, the above configuration with the plurality of coil parts does not interfere with the dilation or deflation of the balloon.
<2> In the second aspect of the present invention, the large-diameter coil part, the small-diameter coil part, and the transitional coil part are formed of the coils of the single, seamless strand. This reduces a loss in the transmission of torque, making it possible to realize higher torque transmissibility.
<3> In the third aspect of the present invention, the large-diameter coil part, the small-diameter coil part, and the transitional coil part are formed of the stranded coil including the plurality of the strands. This can realize higher torque transmissibility.
<4> In the fourth aspect of the present invention, the flexible large-diameter coil part having high torque transmissibility is arranged at the front side of the outer shaft. Furthermore, the highly stiff metal tubular member having higher torque transmissibility is used for the rear side of the outer shaft. With this configuration, the balloon catheter becomes more flexible toward the front end thereof. Therefore, the torque transmissibility can be further improved.
<5> In the fifth aspect of the present invention, the interval between the adjacent coils of the strand constituting the small-diameter coil part is increased. Therefore, the extension part of the inner shaft can be made flexible, although the coil part is arranged thereon.
<6> In the sixth aspect of the present invention, the interval between the adjacent coils of the strand constituting the small-diameter coil part becomes larger toward the front end of the extension part of the inner shaft. This allows a change in flexibility in the range of the extension part. That is, the balloon catheter can be made more flexible toward the front end thereof.
<7> In the seventh aspect of the present invention, the coils of the strand constituting the small-diameter coil part are arranged over substantially the entire length of the extension part of the inner shaft inside the balloon. Therefore, the pushing force or rotational force transmitted from the outer shaft can reliably be transmitted to the front end of the balloon catheter.
<8> In the eighth aspect of the present invention, the interval between the adjacent coils of the strand constituting the transitional coil part is increased. In addition, the interval formed between these coils of the strand serves as the supply hole through which the liquid for dilation is supplied to the balloon. This can realize the simple configuration with little loss in the transmission of torque.

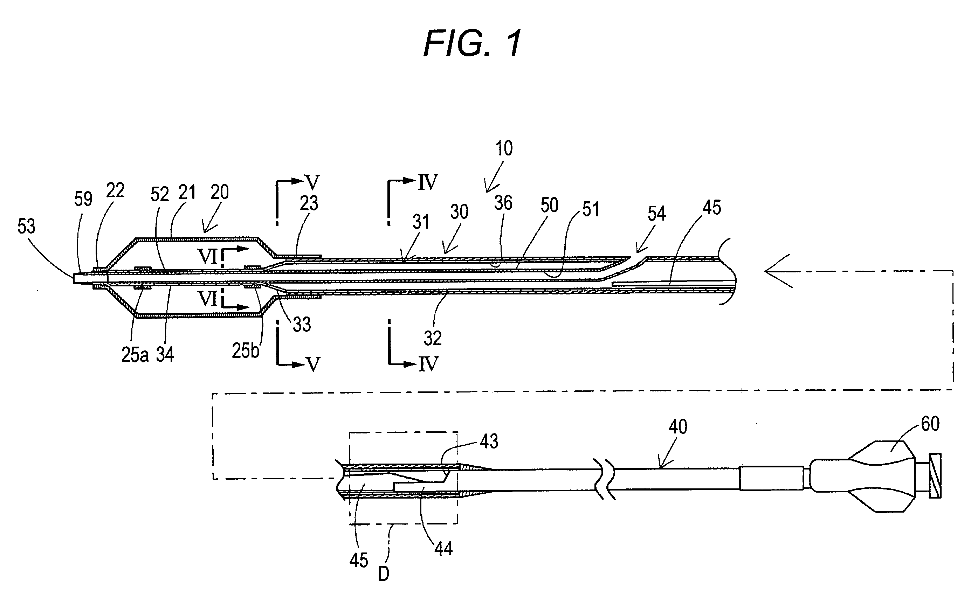

The balloon catheter according to the present embodiment will be described with reference to Figs. 1 to 6.

In Figs. 1 to 3, the left side shown is the front side (distal side) to be inserted into a body, and the right side is the rear side (proximal side, base end side) to be operated by an operator such as a physician.

A balloon catheter 10 is used, for example, to treat an occlusion, a stenosis, or the like of a vessel in the heart. The entire length of the balloon catheter 10 is about 1500 mm.

The balloon catheter 10 mainly includes a balloon 20, a front side outer shaft 30, a stranded coil 31, a rear side outer shaft 40, an inner shaft 50, and a connector 60.

The front side outer shaft 30 is a flexible cylindrical member formed by inserting the stranded coil 31 into a resin tube 38 and then thermally welding the resin tube 38 to the stranded coil 31. In the present embodiment, an outer diameter and an inner diameter of the front side outer shaft 30 are about 0.84 mm and about 0.78 mm, respectively.

Note that the front side outer shaft 30 may also be formed by using a heat-shrinkable tube as the resin tube 38, and closely attaching the resin tube 38 to the stranded coil 31 through the shrinking effect caused by heat.

Examples of the material for the resin tube 38 include resin such as polyamide, polyamide elastomer, polyolefin, polyester, and polyester elastomer.

The stranded coil 31 includes a large-diameter coil part 32, a transitional coil part 33, and a small-diameter coil part 34. The large-diameter coil part 32 is covered with the resin tube 38. The transitional coil part 33 extends from the front end of the resin tube 38, and has a diameter gradually reduced toward the front end thereof. The small-diameter coil part 34 is arranged on an outer circumference of an extension part 52, described later, of the inner shaft 50.

The stranded coil 31 is manufactured in such a manner that a plurality of metal strands 31 a are spirally wound around a mandrel so as to be adjacent to each other, after which the residual stress caused by winding is eliminated by a known heat treatment and then the mandrel is removed. The diameter of the large-diameter coil part 32 thus manufactured is reduced by swaging or the like to obtain the transitional coil part 33 and the small-diameter coil part 34.

Alternatively, the stranded coil 31 may be manufactured using a mandrel having a small-diameter part, a tapered part, and a large-diameter part, which correspond to the small-diameter coil part 34, the transitional coil part 33, and the large-diameter coil part 32, respectively.

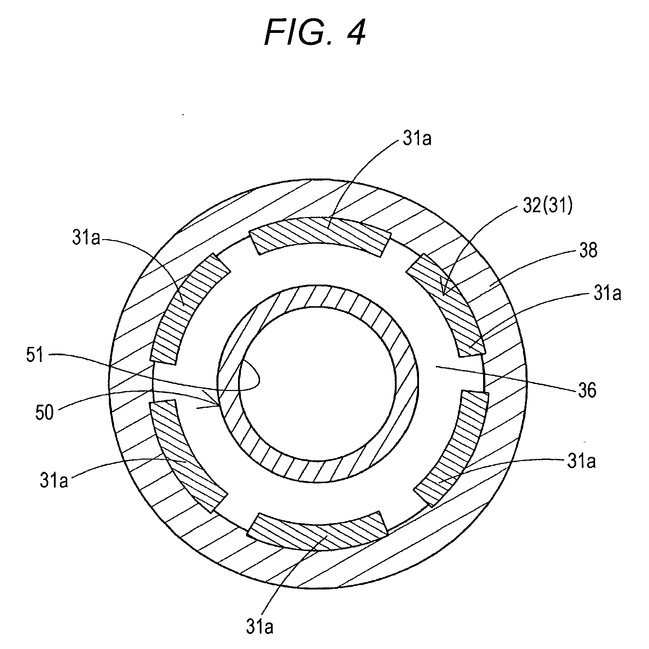

As illustrated in Fig. 4, six strands 31 a are used for the stranded coil 31 of the present embodiment. The strand 31 a is a so-called flat wire having a substantially rectangular cross section. The number and size of the strand 31 a are appropriately determined in view of the outer and inner diameters of and stiffness required for the front side outer shaft 30. Therefore, the number of the strand 31 a is not limited to six. The strand 31 a may also be a round wire having a circular cross section.

The material for the strand 31 a is not particularly limited, but stainless steel is used in the present embodiment. Other materials that may be used include a super elastic alloy such as an Ni-Ti alloy. Alternatively, a plurality of the strands, each made of different materials, may be used in combination.

Referring to Fig. 4, there are seemingly wide intervals between the adjacent strands 31 a because Fig. 4 illustrates the cross section of the strand 31 a as if cut along the cross section perpendicular to the axial direction of the strands 31 a themselves. However, in fact the intervals merely seem to exist. The plurality of the strands 31 a constituting the stranded coil 31 of the front side outer shaft 30 are closely stranded so as to substantially come into contact with each other. As a result, there are only extremely small intervals between the adjacent strands 31 a.

The inner shaft 50 is arranged inside, and coaxially with, the front side outer shaft 30. The inner shaft 50 is a cylindrical member formed by using the same resin as the resin tube 38 of the front side outer shaft 30. The inner shaft 50 includes therein a guidewire lumen 51 through which the guidewire is inserted. The space between an inner circumferential surface of the front side outer shaft 30 and an outer circumferential surface of the inner shaft 50 constitutes a front side dilating lumen 36, through which liquid such as a contrast agent or saline for dilating the balloon 20 flows.

A rear end of the inner shaft 50 is connected to a side surface of the front side outer shaft 30, thereby forming a rear side guidewire port 54. The rear side guidewire port 54 is formed by connecting the rear end of the inner shaft 50 to an inner periphery of a hole bored through the resin tube 38 and the stranded coil 31 by laser. At this time, the strands 31 a of the stranded coil 31 are cut at the bored portion. However, the adjacent strands 31 a are bonded to each other by laser welding, and thus not separated from each other. Therefore, even after the rear side guidewire port 54 is formed, deterioration in the torque transmissibility of the stranded coil 31 can be prevented as much as possible.

The inner shaft 50 has, at the front end thereof, the extension part 52 extending from the front end of the front side outer shaft 30. The extension part 52 has a tip 59 at a front end thereof.

The tip 59 is a member having a tapered outer shape, with an outer diameter gradually reduced toward the front end thereof, and is made of a flexible resin. The tip 59 is a cylindrical member constituting a front end portion of the guidewire lumen 51 and has a front side guidewire port 53 at a front end thereof.

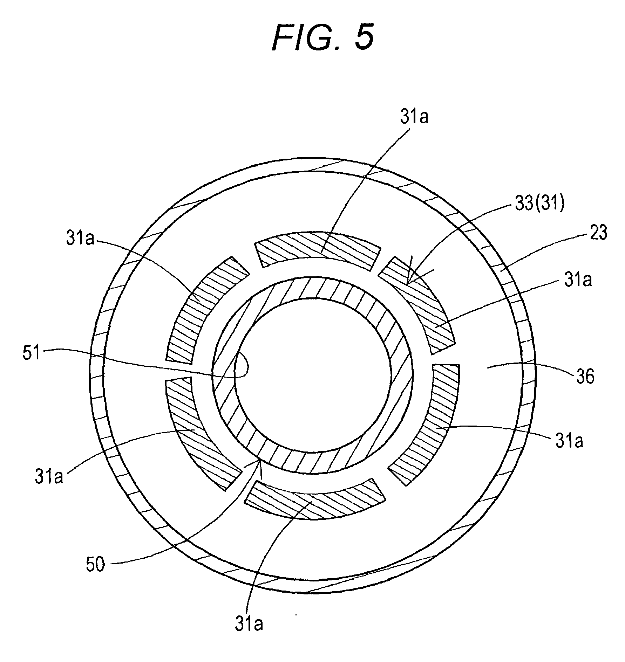

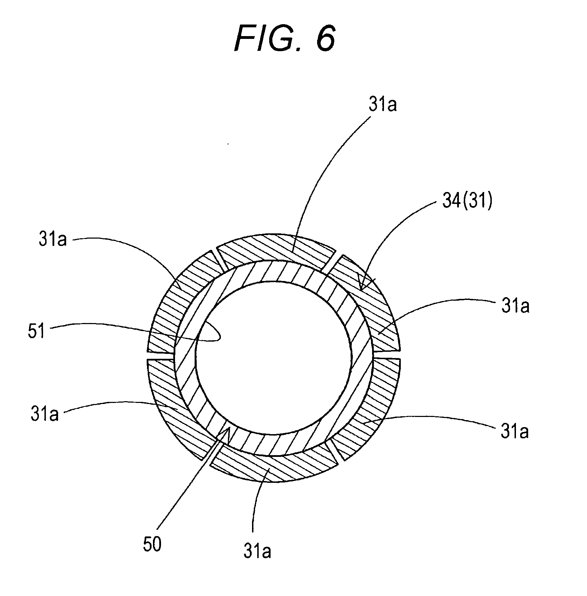

The above-described transitional coil part 33 of the stranded coil 31 is formed between the large-diameter coil part 32 and the small-diameter coil part 34, and has an outer diameter gradually reduced toward the frond side (Fig. 5). The stranded coil 31 extended from the transitional coil part 33 is arranged on the outer circumference of the extension part 52 of the inner shaft 50 as the small-diameter coil part 34 (Fig. 6). The coils of the strand 31a constituting the front end of the small-diameter coil part 34 reach the rear end of the tip 59.

The balloon 20 is a member made of resin. The balloon 20 has a dilated part 21 for dilating the balloon 20, at the center in the axial direction thereof. The balloon 20 also has a front end attachment part 22 and a rear end attachment part 23 at the front and rear sides thereof, respectively.

The front end attachment part 22 is firmly attached to the front end portion of the extension part 52 of the inner shaft 50. In the present embodiment, the front end attachment part 22 is firmly attached to the outer circumferential surface of the tip 59. Furthermore, the front end attachment part 22 surrounds the coils of the strands 31 a of the front end of the small-diameter coil part 34, thereby bonding the coils of the strands 31 a to the tip 59.

The rear end attachment part 23 is firmly attached to the outer circumferential surface of the front end of the front side outer shaft 30.

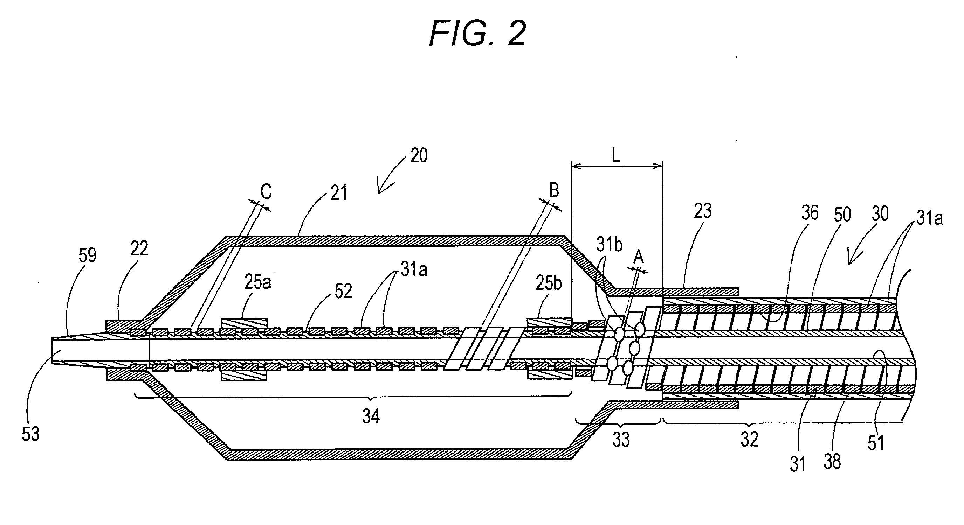

As illustrated in Fig. 2, an interval between the adjacent coils of the strands 31 a is increased in the transitional coil part 33 and the small-diameter coil part 34 of the stranded coil 31. Therefore, a gap is formed between the adjacent coils of the strands 31a. In the present embodiment, an interval A between the coils of the strands 31a in the transitional coil part 33 is set smaller than an interval B between the coils of the strands 31a in the small-diameter coil part 34. However, the interval A between the coils of the strands 31a in the transitional coil part 33 serves as a supply hole through which liquid for dilating the balloon 20 flows into the balloon 20, the liquid being supplied through the front side dilating lumen 36 of the front side outer shaft 30. Therefore, the interval A needs to be wide enough to flow the liquid for dilation.

In addition, in order to prevent the unnecessary increase in the interval between the coils of the strands while maintaining the interval A between the coils of the strands 31 a in the transitional coil part 33, the adjacent coils of the strands 31 a in the transitional coil part 33 are bonded to each other by laser welding. The laser welding results in the formation of a laser-welded part 31 b.

In the present embodiment, a length L of the transitional coil part 33 in the axial direction of the inner shaft 50 is set to about 3.0 mm.

Also in the present embodiment, as illustrated in Fig. 2, the front end of the transitional coil part 33 is positioned in the dilated part 21 of the balloon 20. However, the front end of the transitional coil part 33 may be positioned outside the dilated part 21 of the balloon 20 by extending the axial length of the rear end attachment part 23 of the balloon 20, in which case an outer diameter of the folded balloon 20 can be reduced.

The flexibility of the small-diameter coil part 34 of the stranded coil 31 is improved due to the interval B between the coils of the strand 31 a. That is, the presence of the interval B prevents the unnecessary increase in the stiffness of the extension part 52 of the inner shaft 50, which would otherwise be caused by the stranded coil 31. At the same time, the interval B enables the torque, transmitted from the large-diameter coil part 32 of the stranded coil 31 via the transitional coil part 33, to be transmitted to the inner shaft 50 and the tip 59.

Note that the interval between the strands 31a in Fig. 5 is illustrated to be smaller than that in Fig. 4, and even smaller in Fig. 6. This is because, as in Fig. 4, Figs. 5 and 6 each illustrate the cross section of the strands 31 a in the cross section of the stranded coil 31 whose diameter has been reduced, as if cut along the cross section perpendicular to the axial direction of the strands 31 a. As described above, in fact the interval between the adjacent coils of the strands 31 a in the portions illustrated in Figs. 5 and 6 in the axial direction of the balloon catheter 10 is larger than that in the portion illustrated in Fig. 4.

In the present embodiment, the interval B between the coils of the strands 31a in the small-diameter coil part 34 is constant. That is, when the interval between the coils of the strands 31 a at the rear side of the small-diameter coil part 34 is defined as B while the interval at the front side thereof as C, the interval B has the same width as the interval C. However, the interval between the coils of the strands 31 a of the small-diameter coil part 34 may be changed by, for example, increasing the interval toward the front end. That is, the small-diameter coil part 34 can be made more flexible toward the front end thereof, in such a manner that the width of the interval C between the coils of the strands 31 a at the front side of the extension part 52 is made larger than the width of the interval B between the coils of the strands 31 a at the rear side of the extension part 52.

A pair of radiopaque markers 25a and 25b, spaced apart from each other by a predetermined distance, is attached to the portion of the extension part 52 of the inner shaft 50 inside the dilated part 21 of the balloon 20.

The rear side outer shaft 40 is a tubular member made of metal, a so-called hypotube, having therein a rear side dilating lumen 46. A tip portion of the rear side outer shaft 40 is inserted into, and firmly attached to, a rear end portion of the front side outer shaft 30. As a result, the rear side dilating lumen 46 is in communication with the front side dilating lumen 36 of the front side outer shaft 30.

The connector 60 is attached to a rear end of the rear side outer shaft 40. An indeflator (not shown) attached to the connector 60 supplies liquid for dilating the balloon 20. Consequently, the liquid flows into the balloon 20 through the rear side dilating lumen 46 and the front side dilating lumen 36, thereby dilating the balloon 20.

In the present embodiment, an outer diameter and an inner diameter of the rear side outer shaft 40 are about 0.62 mm and about 0.48 mm, respectively. The material for the rear side outer shaft 40 is not particularly limited, but stainless steel is used as the material in the present embodiment. Other materials that may be used include a super elastic alloy such as an Ni-Ti alloy.

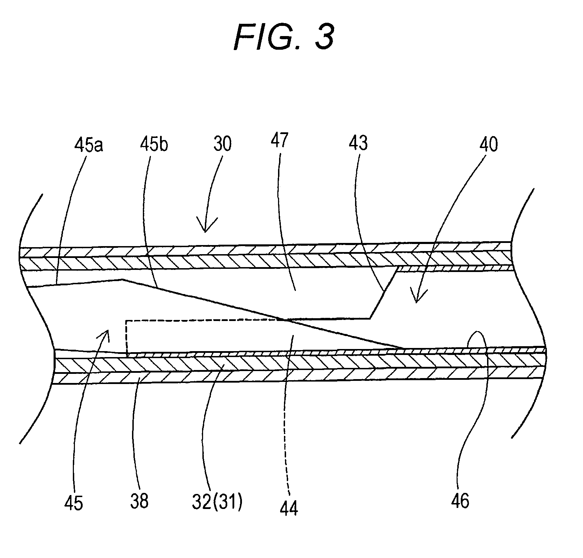

As illustrated in Fig. 3, the tip portion of the rear side outer shaft 40 has an inclined opening part 43 and a protruding part 44. The inclined opening part 43 is formed to be inclined toward the front side, at least down to the middle position in the radial direction. The protruding part 44 is substantially in a half cylindrical shape. The protruding part 44 extends along the axial direction from the inclined opening part 43 toward the front side. That is, the tip portion of the rear side outer shaft 40 is formed by obliquely cutting into the shaft down to the middle position in the radial direction, and then cutting off the upper side of the shaft along the axial direction.

A core wire 45 is attached to the protruding part 44 of the rear side outer shaft 40.

The core wire 45 is inserted into the front side outer shaft 30 and extends to the vicinity of the rear side guidewire port 54. The core wire 45 is made of, for example, stainless steel or a super elastic alloy such as an Ni-Ti alloy.

The core wire 45 has a shaft part 45a and an attachment part 45b. The shaft part 45a is provided on the front side and has a tapered shape with a diameter reduced toward the front end. The attachment part 45b is provided on the rear side and inclined toward the rear end.

The diameter of the shaft part 45a of the core wire 45 is reduced toward the front end. As a result, the shaft part 45a allows a stiffness change in the balloon catheter 10, such that the balloon catheter 10 becomes more flexible toward the front end.

The attachment part 45b of the core wire 45 extends to the rear end of the inclined opening part 43. The attachment part 45b is firmly attached to an inner wall of the protruding part 44 of the rear side outer shaft 40 by brazing, welding using laser, or the like.

With this configuration, a space 47 is formed between the inclined opening part 43 of the rear side outer shaft 40 and the attachment part 45b of the core wire 45. Consequently, the liquid for dilating the balloon, which flows out of the rear side dilating lumen 46 of the rear side outer shaft 40, flows into the front side dilating lumen 36 of the front side outer shaft 30.

Also with this configuration, a stiffness change at a connecting portion of the core wire 45 and the rear side outer shaft 40 is alleviated as much as possible, while the balloon catheter 10 is prevented from bending at the connecting portion.

Note that, in the present embodiment, the shaft part 45a has the tapered shape over the entire length thereof. However, the shaft part 45a may include a part where the diameter is constant, that is, the outer diameter does not change in the axial direction.

The case of using the balloon catheter 10 according to the present embodiment in an operation of dilating a stenosis in a coronary artery of the heart will be described, based on the above configuration.

A guidewire (not shown) is inserted in advance into the coronary artery of the heart, where the stenosis as a target to be treated is located. The balloon catheter 10 is inserted into the body along the guidewire. The guidewire is inserted from the front side guidewire port 53 at the tip 59 of the balloon catheter 10, passes through the guidewire lumen 51 inside the inner shaft 50, and extends from the rear side guidewire port 54.

When advancing the balloon catheter 10 along the guidewire inside a vessel, an operator such as a physician pushes the balloon catheter 10 in the axial direction from the proximal side. This pushing force is transmitted from the rear side outer shaft 40, which is a metal tube, to the front side outer shaft 30. As described above, the front side outer shaft 30 includes the resin tube 38 and the stranded coil 31. Therefore, despite higher flexibility than that of the rear side outer shaft 40, the front side outer shaft 30 can effectively transmit the pushing force to the front end by virtue of the stranded coil 31. Furthermore, the small-diameter coil part 34 of the stranded coil 31, which extends from the front end of the front side outer shaft 30, is arranged at the extension part 52 of the inner shaft 50 via the transitional coil part 33. With this configuration, the pushing force transmitted from the large-diameter coil part 32 of the stranded coil 31 can effectively be transmitted to the tip 59, which is the front end of the balloon catheter 10.

The advance of the balloon catheter may be blocked at a tortuous part or the like of the vessel. In this case, the operator turns the balloon catheter 10 around the axis thereof from the proximal side. This imparts a rotational force to the balloon catheter 10. As a result, as in the case of the above pushing force, the rotational force is effectively transmitted to the large-diameter coil part 32, the transitional coil part 33, and then to the small-diameter coil part 34 by the stranded coil 31, thereby allowing the tip 59 to turn. In this case, the front end portion of the catheter near the tip 59 is supposed to turn by about 45 to 90 degrees. In this manner, the balloon catheter 10 is configured to favorably transmit the rotational force to the front end portion thereof. With this configuration, the turning of the front end portion enables the balloon catheter 10 to advance smoothly, even when the advance of the balloon catheter 10 is blocked once at a tortuous vessel or the like.

Meanwhile, the interval between the adjacent coils of the strands 31 a is increased in the small-diameter coil part 34 of the stranded coil 31, which coil part is located at the front end portion of the balloon catheter 10. This improves the flexibility of the front end portion and, therefore, prevents the flexibility of the front end portion of the balloon catheter 10 from being undesirably impaired by the stranded coil 31.

The operator positions the balloon 20 at the stenosis as a target site, using the markers 25a and 25b under radioscopy. Subsequently, the indeflator (not shown) attached to the connector 60 supplies the liquid for dilation, such as a contrast agent or saline.

At this time, the liquid for dilation flows into the front side dilating lumen 36 of the front side outer shaft 30 from the rear side dilating lumen 46 of the rear side outer shaft 40. The liquid for dilation then flows out of the interval A between the coils of the strands 31 a provided at the transitional coil part 33 of the stranded coil 31, thereby dilating the balloon 20. The transitional coil part 33 is provided at the front end of the front side outer shaft 30.

Upon complete operation of dilating the stenosis with the balloon 20, the operator uses the indeflator to discharge the liquid for dilation out of the balloon 20. That is, the liquid for dilation exits the balloon 20 and then flows out of the interval A between the coils of the strands 31 a provided at the transitional coil part 33 of the stranded coil 31, and is discharged through the front side dilating lumen 36 of the front side outer shaft 30 and the rear side dilating lumen 46 of the rear side outer shaft 40.

As described above, the front side outer shaft 30 of the balloon catheter 10 according to the present embodiment has the large-diameter coil part 32, which improves the torque transmissibility. Furthermore, the inner shaft 50 has arranged thereon the small-diameter coil part 34 formed by reducing the diameter of the distal portion of the stranded coil 31 constituting the large-diameter coil part 32. Consequently, the pushing force or rotational force transmitted from the front side outer shaft 30 is transmitted to the extension part 52 of the inner shaft 50, and further to the front end of the balloon catheter 10. Therefore, the balloon 20 does not block the transmission of the torque from the front side outer shaft 30, making it possible to exhibit high torque transmissibility.

Meanwhile, the flexibility of the extension part 52 of the inner shaft 50, where the balloon 20 is located, can be maintained by adjusting the interval between the coils of the strands 31 a of the small-diameter coil part 34.

The outer shaft of the balloon catheter 10 has the rear side outer shaft 40 formed of a metal tube and the front side outer shaft 30 including the resin tube 38 and the stranded coil 31. Therefore, the outer shaft of the balloon catheter 10 has the property of a metal tube with extremely high torque transmissibility, in combination with the property of the stranded coil 31 having both flexibility and high torque transmissibility. As a result, the balloon catheter 10 has a structure in which the flexibility and torque transmissibility become higher toward the front end thereof.

In the embodiment described above, the outer shaft of the balloon catheter 10 is divided into the front side outer shaft 30 and the rear side outer shaft 40. The stranded coil 31 of the large-diameter coil part 32 is provided only in the front side outer shaft 30. However, the stranded coil may be provided over the entire length of the outer shaft.

Also in the embodiment described above, the stranded coil 31 of the small-diameter coil part 34 is provided over substantially the entire length of the extension part 52 of the inner shaft 50. This configuration is preferable in terms of sufficiently transmitting the torque to the front end of the balloon catheter 10. Alternatively, the stranded coil 31 may be provided only at a part of the extension part 52 of the inner shaft 50 in order to, for example, improve the flexibility of the extension part 52 of the inner shaft 50.

In the embodiment described above, the interval A between the coils of the strands 31 a of the transitional coil part 33 and the interval B between the coils of the strands 31 a of the small-diameter coil part 34 are formed by widening the interval between the coils of the strand 31 a. Alternatively, however, the intervals A and B may be formed by decreasing the number of the strands 31 a constituting the transitional coil part 33 and the small-diameter coil part 34. In this case, the cutting portions of the coils of the strands 31 a are preferably subjected to laser welding or the like so as not to be separated from each other.

The method for improving the flexibility of the transitional coil part 33 and the small-diameter coil part 34 is not limited to the one described above. For example, the stiffness of the coils of the strands 31 a may be lowered in such a manner that centerless grinding, electrolytic polishing or the like is performed on the coils of the strands 31 a of the small-diameter coil part 34 to thereby reduce the thickness of the coils of the strands. Also with this method, the flexibility of the transitional coil part 33 and the small-diameter coil part 34 can be improved. Further alternatively, the stiffness and flexibility of the transitional coil part 33 and the small-diameter coil part 34 can be adjusted by increasing or decreasing the number of the laser-welded parts 31 b.

In the embodiment described above, the interval A between the coils of the strand 31 a serves as the supply hole of the transitional coil part 33. Alternatively, however, the supply hole may be formed by boring a hole in the transitional coil part 33 with laser or the like, while maintaining the close contact between the adjacent coils of the strands. Also in this case, the coils of the strands 31 a around the bored hole are preferably subjected to laser welding or the like, so as not to be separated from each other.

In the embodiment described above, the balloon catheter 10 has a so-called rapid exchange type in which the guidewire lumen 51 is short, by forming the rear side guidewire port 54 on the side surface of the front side outer shaft 30. Alternatively, however, the balloon catheter 10 may have a so-called over-the-wire type in which the inner shaft 50 extends to the rear end of the balloon catheter 10.

In the embodiment described above, the large-diameter coil part 32, the transitional coil part 33, and the small-diameter coil part 34 are formed of the plurality of the single, seamless strands 31 a as one stranded coil 31 having different diameters depending on the portions thereof. Alternatively, however, the large-diameter coil part 32, the transitional coil part 33, and the small-diameter coil part 34 may be formed as separate coils, each having different diameters. In this case, the separate coils may be connected by laser welding or the like. When the small-diameter coil part 34 is formed of coils made of radiopaque metal, for example, the markers 25a and 25b are no longer necessary.

Nonetheless, to improve the torque transmissibility as much as possible, the large-diameter coil part 32, the transitional coil part 33, and the small-diameter coil part 34 are preferably formed of the plurality of single, seamless strands 31 a, as in the embodiment described above.

In the embodiment described above, the large-diameter coil part 32, the transitional coil part 33, and the small-diameter coil part 34 are formed of the stranded coil 31 including the plurality of the strands 31 a. Alternatively, however, the stranded coil 31 may be formed of a single strand. Alternatively, only a part of the three coil parts may be formed of the single strand. Nonetheless, to improve the torque transmissibility as much as possible, each coil part is preferably formed of the stranded coil including the plurality of the strands, as in the embodiment described above.

In the embodiment described above, the balloon catheter 10 is used for the treatment of a vessel in the heart. However, the use of the balloon catheter 10 is not limited to such treatment. The balloon catheter 10 can be used for various operations such as the treatment of a vessel in the lower limb, and the operation of dilating a dialysis shunt.

## Claims

1. A balloon catheter comprising:
a balloon (20);
a tubular outer shaft (30) connected to a rear end attachment part (23) of the balloon (20);
an inner shaft (50) inserted into the outer shaft (30) and having an extension part (52) extending from a front end of the outer shaft (30) into the balloon (20), the extension part (52) being connected to a front end attachment part (22) of the balloon (20);
a small-diameter coil part (34) including at least one strand wound around at least a part of the extension part (52) of the inner shaft (50); and
a transitional coil part (33) including at least one strand which is connected to the small-diameter coil part (34), **characterized by**:
a large-diameter coil part (32) including at least one strand wound within at least a part of the outer shaft (30),
wherein the transitional coil part (33) connects the large-diameter coil part (32) and the small-diameter coil part (34) and has a supply hole through which liquid for dilating the balloon (20) is supplied.

2. The balloon catheter according to claim 1, wherein each strand of the large-diameter coil part (32), the small-diameter coil part (34), and the transitional coil part (33) is formed of a single, seamless strand.

3. The balloon catheter according to claim 1 or 2, wherein the large-diameter coil part (32), the small-diameter coil part (34), and the transitional coil part (33) are formed of a stranded coil including a plurality of strands.

4. The balloon catheter according to any one of claims 1 to 3, wherein the large-diameter coil part (32) is arranged at a front side (30) of the outer shaft (30), and
a rear side (40) of the outer shaft (30) is formed of a metal tubular member.

5. The balloon catheter according to any one of claims 1 to 4, wherein an interval between adjacent coils of the strand constituting the small-diameter coil part (34) is larger than an interval between adjacent coils of the strand constituting the large-diameter coil part (32).

6. The balloon catheter according to claim 5, wherein the interval between the adjacent coils of the strand constituting the small-diameter coil part (34) becomes larger toward a front end of the extension part (52) of the inner shaft (50).

7. The balloon catheter according to any one of claims 1 to 6, wherein coils of the strand constituting the small-diameter coil part (34) reach a front end portion of the extension part (52) of the inner shaft (50), the front end portion of the extension part (52) being connected to the front end attachment part (22) of the balloon (20).

8. The balloon catheter according to any one of claims 1 to 7, wherein an interval between adjacent coils of the strand constituting the transitional coil part (33) is larger than an interval between adjacent coils of the strand constituting the large-diameter coil part (32), and the interval formed between the adjacent coils of the strand in the transitional coil part (33) forms the supply hole.

## Patentansprüche

1. Ballonkatheter mit:
einem Ballon (20);
einem mit einem hinteren Ansatz (23) des Ballons (20) verbundenen rohrförmigen Außenschaft (30);
einem im Außenschaft (30) aufgenommenen Innenschaft (50) mit einer Verlängerung (52), die sich vom vorderen Ende des Außenschafts (30) in den Ballon (20) hinein erstreckt und mit einem vorderen Ansatz (22) des Ballons (20) verbunden ist;
einem im Durchmesser kleinen Wicklungsteil (34) aus wenigstens einem Strang, der um wenigstens einen Teil der Verlängerung (52) des Innenschafts (50) gewickelt ist; und
einem Übergangswicklungsteil (33) aus wenigstens einem Strang, der mit dem im Durchmesser kleinen Wicklungsteil (34) verbunden ist, **gekennzeichnet durch**:
einen im Durchmesser großen Wicklungsteil (32) aus wenigstens einem Strang, der um wenigstens einen Teil des Außenschafts (30) gewickelt ist,
wobei der Übergangswicklungsteil (33) den im Durchmesser großen Wicklungsteil (32) und den im Durchmesser kleinen Wicklungsteil (34) verbindet und eine Zufuhröffnung aufweist, **durch** welche Flüssigkeit zum Dehnen des Ballons (20) zugeführt wird.

2. Ballonkatheter nach Anspruch 1, wobei jeder Strang des im Durchmesser großen Wicklungsteils (32), des im Durchmesser kleinen Wicklungsteils (34) und des Übergangswicklungsteils (33) aus einem einzelnen nahtlosen Strang gebildet ist.

3. Ballonkatheter nach Anspruch 1 oder 2, wobei der im Durchmesser große Wicklungsteil (32), der im Durchmesser kleine Wicklungsteil (34) und der Übergangswicklungsteil (33) aus einer mehrsträngigen Wicklung gebildet sind.

4. Ballonkatheter nach einem der Ansprüche 1 bis 3, wobei
der im Durchmesser große Wicklungsteil (32) an einer Vorderseite (30) des Außenschafts (30) angeordnet ist, und
eine Rückseite (40) des Außenschafts (30) aus einem Metallrohrteil gebildet ist.

5. Ballonkatheter nach einem der Ansprüche 1 bis 4, wobei der Abstand zwischen nebeneinander liegenden Strangwindungen, die den im Durchmesser kleinen Wicklungsteil (34) bilden, größer ist als der Abstand zwischen nebeneinander liegenden Strangwindungen, die den im Durchmesser großen Wicklungsteil (32) bilden.

6. Ballonkatheter nach Anspruch 5, wobei der Abstand zwischen den nebeneinander liegenden Strangwindungen, die den im Durchmesser kleinen Wicklungsteil (34) bilden, zum vorderen Ende der Verlängerung (52) des Innenschafts (50) hin größer wird.

7. Ballonkatheter nach einem der Ansprüche 1 bis 6, wobei
die Strangwindungen, die den im Durchmesser kleinen Wicklungsteil (34) bilden, sich bis zum vorderen Endabschnitt der Verlängerung (52) des Innenschafts (50) erstrecken, und
der vordere Endabschnitt der Verlängerung (52) mit dem vorderen Ansatz (22) des Ballons (20) verbunden ist.

8. Ballonkatheter nach einem der Ansprüche 1 bis 7, wobei
der Abstand zwischen nebeneinander liegenden Strangwindungen, die den Übergangswicklungsteil (33) bilden, größer ist als der Abstand zwischen nebeneinander liegenden Strangwindungen, die den im Durchmesser großen Wicklungsteil (32) bilden, und
der Abstand zwischen den nebeneinander liegenden Strangwindungen des Übergangswicklungsteils (33) die Zufuhröffnung bildet.

## Revendications

1. Cathéter à ballonnet comprenant :
un ballonnet (20) ;
un tube externe (30) relié à une pièce de fixation à l'extrémité arrière (23) du ballonnet (20) ;
une tige interne (50) insérée dans le tube externe (30) et ayant un segment d'extension (52) s'étendant depuis l'extrémité avant du tube externe (30) jusque dans le ballonnet (20), le segment d'extension (52) étant relié à une pièce de fixation à l'extrémité avant (22) du ballonnet (20) ;
une partie bobine de petit diamètre (34) incluant au moins un brin enroulé autour au moins d'une partie du segment d'extension (52) de la tige interne (50) ; et
une partie à inductance de passage (33) incluant au moins un brin qui est relié à la partie bobine de petit diamètre (34), **caractérisé par** :
une partie bobine de grand diamètre (32) incluant au moins un brin enroulé autour au moins d'une partie du tube externe (30),
dans lequel la partie à inductance de passage (33) se raccorde à la partie bobine de grand diamètre (32) et à la partie bobine de petit diamètre (34) et comprend un trou d'alimentation au travers duquel le liquide servant à dilater le ballonnet (20) est fourni.

2. Cathéter à ballonnet selon la revendication 1, dans lequel chaque brin de la partie bobine de grand diamètre (32), de la partie bobine de petit diamètre (34), et de la partie à inductance de passage (33) est formé à partir d'un unique brin sans soudure.

3. Cathéter à ballonnet selon la revendication 1 ou 2, dans lequel la partie bobine de grand diamètre (32), la partie bobine de petit diamètre (34), et la partie à inductance de passage (33) sont formées à partir d'une bobine à brins comprenant une pluralité de brins.

4. Cathéter à ballonnet selon l'une quelconque des revendications 1 à 3, dans lequel la partie bobine de grand diamètre (32) est disposée du côté avant (30) du tube externe (30), et un côté arrière (40) du tube externe (30) est formé à partir d'un élément métallique tubulaire.

5. Cathéter à ballonnet selon l'une quelconque des revendications 1 à 4, dans lequel un intervalle entre les bobines adjacentes du brin constituant la partie bobine de petit diamètre (34) est plus grand qu'un intervalle entre les bobines adjacentes du brin constituant la partie bobine de grand diamètre (32).

6. Cathéter à ballonnet selon la revendication 5, dans lequel l'intervalle entre les bobines adjacentes du brin constituant la partie bobine de petit diamètre (34) devient plus grand vers l'extrémité avant du segment d'extension (52) de la tige interne (50).

7. Cathéter à ballonnet selon l'une quelconque des revendications 1 à 6, dans lequel les bobines du brin constituant la partie bobine de petit diamètre (34) atteignent la portion à l'extrémité avant du segment d'extension (52) de la tige interne (50), la portion à l'extrémité avant du segment d'extension (52) étant reliée à une pièce de fixation à l'extrémité avant (22) du ballonnet (20).

8. Cathéter à ballonnet selon l'une quelconque des revendications 1 à 7, dans lequel un intervalle entre les bobines adjacentes du brin constituant la partie à inductance de passage (33) est plus grand qu'un intervalle entre les bobines adjacentes du brin constituant la partie bobine de grand diamètre (32), et l'intervalle formé entre les bobines adjacentes du brin dans la partie à inductance de passage (33) forme le trou d'alimentation.
